# FASCICULE DE BREVET EUROPEEN

(11) **EP 3 352 860 B1**
(45) Date de publication et mention de la délivrance du brevet: **15.07.2020**
(21) Numéro de dépôt: 16770494.9
(22) Date de dépôt: 22.09.2016
(51) Int. Cl.: A61Q 19/00, A61K 8/31

(54) **INGREDIENT COSMETIQUE A BASE D'HYDROCARBURES SATURES RAMIFIES**
KOSMETISCHER INHALTSSTOFF MIT VERZWEIGTEM GESÄTTIGTEM KOHLENWASSERSTOFF
BRANCHED SATURATED HYDROCARBON COSMETIC INGREDIENT

(30) Priorité: 22.09.2015 FR 1558954
(43) Date de publication de la demande: 01.08.2018
(73) Titulaire: Biosynthis, 91410 Saint Cyr Sous Dourdan (FR)
(72) Inventeur: BERNOUD, Thierry, 91410 Saint Cyr sous Dourdan (FR); MAGNE, Julien, 86340 Roches-Premarie-Andille (FR); PICCIRILLI, Antoine, 86000 Poitiers (FR)
(74) Mandataire: Tripoz, Inès
(86) Numéro de dépôt international: PCT/EP2016/072621
(87) Numéro de publication internationale: WO 2017/050943

(56) Documents cités:
- WO-A1-2005/067892
- WO-A1-2006/014035
- WO-A1-2012/059702
- WO-A1-2013/093411
- DE-A1- 4 124 300
- FR-A1- 2 235 897
- FR-A1- 2 250 515
- US-A- 3 886 287

## Description

Le squalane (C₃₀H₆₂), 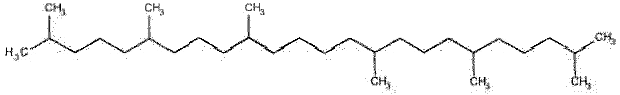 obtenu par hydrogénation du squalène (C₃₀H₅₀), 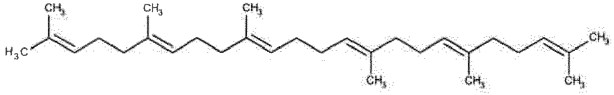 est un ingrédient cosmétique très utilisé notamment en raison des caractéristiques sensorielles très particulières qu'il confère aux compositions dans lesquelles il est intégré, de ses qualités techniques exceptionnelles en terme de formulation et de ses propriétés dermatologiques. A l'origine il était obtenu à partir de squalène extrait de l'huile de foies de requins puis hydrogéné.
Un squalane végétal de substitution a ensuite été extrait de l'huile d'olive mais ce squalane d'olive souffre notamment de difficultés d'approvisionnement en raison de problèmes climatiques, des maladies et des parasites qui s'attaquent aux oliviers dans le monde entier. En 2014 par exemple la production européenne a subi une baisse de 45 % principalement en raison de problèmes climatiques. En 2015, la prolifération de *Xylella fastidiosa* entraîne une menace sur la production italienne.

Les procédés d'élimination des cires (winterisation) et de purification entraînent également des pertes de 10 à 20 %.

De plus l'évolution des procédés de raffinage de l'huile d'olive et la mise en œuvre de procédés à des températures élevées favorisent la formation de sous-produits tels que des isomères et des produits cycliques qui entraînent des variations dans la qualité des squalènes extraits d'huile d'olive.

De nombreux produits de substitution ont été recherchés pour répondre à une demande toujours croissante et des substituts d'origines végétale diverses par exemple la canne à sucre ont été préparés. On citera par exemple, un squalane végétal dit squalane « squalane de sucre », obtenu par la condensation du farnésène par des procédés de fermentation cependant les propriétés, notamment celles dues aux constituants minoritaires que sont les phytostérols et les cires végétales du squalane d'olive ne sont pas toutes retrouvées.

La plupart des substituts synthétisés pour imiter la structure du squalane sont des alcanes qui comportent de nombreuses ramifications et des substituants méthyles ou éthyles, entrainant l'obligation d'avoir recours à des synthèses impossible à mettre en œuvre à partir de composés d'origine naturelle et végétale. C'est le cas notamment des composés décrits dans le brevet US 3886287 au nom de SHISEIDO qui, s'ils satisfont au cahier des charges relatif à la tolérance et ont une haute stabilité vis-à-vis de l'action des microorganismes, ne sont pas comparés d'un point de vue sensoriel au squalane.

De nombreuses autres sources végétales telles que le tournesol ou la cire de riz sont exploitées régulièrement sans que les propriétés organoleptiques ou sensorielles du squalane d'olive puissent être reproduites.

De façon surprenante, un ingrédient cosmétique comprenant au moins un alcane en C24 à C48, sous forme au moins dimérique, peut-être avantageusement substitué à un squalane végétal dans des formulations cosmétiques tout en conservant, voire en améliorant les caractéristiques sensorielles dudit squalane végétal.

On entend par sous forme dimérique un composant résultant de la combinaison (dimérisation) de deux molécules.

De plus ces caractéristiques sont atteintes avec des alcanes qui ne comportent pas de nombreux substituants à chaines courtes comme des méthyles ou des éthyles, donc sans chercher à mimer la structure polysubstituée par des radicaux méthyle du squalane végétal.

Dans un mode de réalisation, le squalane végétal est un squalane d'olive.

L'ingrédient selon l'invention ajouté au squalane végétal n'altère pas les propriétés du squalane végetal et il peut être ajouté dans des proportions comprises entre 10 et 80 %.

Dans un mode de réalisation il peut être ajouté dans des proportions comprises entre 10 et 50 %.

On a ainsi la possibilité de diminuer de façon importante la quantité de squalane végetal sans aucune conséquence négative sur les propriétés des produits qui seront formulés.

L'invention concerne un ingrédient cosmétique comprenant au moins un squalane végétal et au moins un mélange d'alcanes en C24 à C48, sous forme au moins dimérique, choisis parmi les alcanes de formule I, 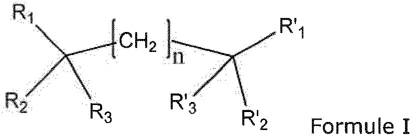 dans laquelle
- n est égal à 1 ou 0,
- R₁, R'₁, R₂, R'₂, R₃ et R'₃ identiques ou différents sont choisis dans le groupe constitué par les atomes d'hydrogène -H, les radicaux méthyles et les radicaux alkyles linéaires en C8 à C30,
- au moins un des R₁, R'₁, R₂, R'₂, R₃ et R'₃ identiques ou différents est choisi parmi les radicaux alkyles, linéaires ou ramifiés en C8 à C30,
et l'un au moins et l'un au plus des R₁, R'₁, R₂, R_{'2} R₃ et R'₃ est un radical méthyle, ledit mélange d'alcanes en C24 à C48 ayant une viscosité comprise entre 12 et 25 mm²/s à 40°C.

L'invention concerne un ingrédient cosmétique comprenant au moins un squalane végétal et au moins un mélange d'alcanes en C24 à C48, sous forme au moins dimérique, choisis parmi les alcanes de formule I, 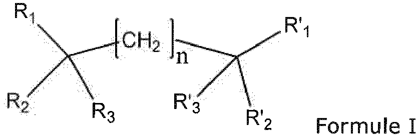 dans laquelle
- n est égal à 1 ou 0,
- au moins un des R₁, R'₁, R₂, R_{'2}, R₃ et R'₃ identiques ou différents est choisi dans un groupe constitué par les radicaux alkyles, linéaires ou ramifiés en C8 à C30,
- au moins un des R₁, R₂ et R₃ est un atome d'hydrogène -H,
- au moins un des R'₁, R'₂ et R'₃ est un atome d'hydrogène -H,
et l'un au plus des R₁, R'₁, R₂, R'₂ R₃ et R'₃ est un radical méthyle, ledit mélange d'alcanes en C24 à C48 ayant une viscosité comprise entre 12 et 25 mm²/s à 40°C.

Dans un mode de réalisation, au moins un des R₁, R'₁, R₂, R'₂, R₃ et R'₃ identiques ou différents est choisi parmi les radicaux alkyles linéaires en C8 à C30

Dans un mode de réalisation, R₁, R'₁, R₂, R'₂, R₃ et R'₃ identiques ou différents sont choisis dans le groupe constitué par les atomes d'hydrogène -H, les radicaux méthyles et des radicaux alkyles, linéaires ou ramifiés en C8 à C30.

Dans un mode de réalisation, R₁, R'₁, R₂, R'₂, R₃ et R'₃ identiques ou différents sont choisis dans le groupe constitué par les atomes d'hydrogène -H, les radicaux méthyles et des radicaux alkyles linéaires en C8 à C30.

Dans un mode de réalisation, au moins un des R₁, R₂ ou, R₃ et au moins un des, R'₁, R'₂ ou R'₃ est un atome d'hydrogène.

Dans un mode de réalisation, R'₁ est un radical méthyle.

Dans un mode de réalisation, R'₁ est un radical méthyle, au moins un des R₁, R₂ ou, R₃ et au moins un des, R'₂ ou R'₃ est un atome d'hydrogène -H.

Dans un mode de réalisation, n=0, R'₁ est un radical méthyle, R1, R2 et R'2 sont des atomes d'hydrogène -H et R3 et R'3 sont choisis parmi les radicaux alkyles, linéaires ou ramifiés en C8 à C30 et les alcanes du au moins un mélange d'alcanes en C24 à C48, sous forme au moins dimérique sont choisis parmi les alcanes de formule Ia.

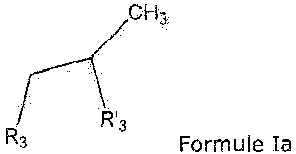

Dans un mode de réalisation, n=0, R'₁ est un radical méthyle, R1, R2 et R'2 sont des atomes d'hydrogène -H et R3 et R'3 sont choisis parmi les radicaux alkyles, linéaires en C8 à C30 et les alcanes du au moins un mélange d'alcanes en C24 à C48, sous forme au moins dimérique sont choisis parmi les alcanes de formule la.

Dans un mode de réalisation, les alcanes ramifiés de formule I et Ia sont obtenus par dimérisation successives d'alcools gras issus de l'hydrogénation catalytique des esters méthyliques de coco, et comprennent des dimères, des trimères, quadrimères ou pentamères, puis déshydratation et hydrogénation.

Dans un mode de réalisation préféré, les dimérisations successives sont obtenues par réaction de Guerbet.

Dans un mode de réalisation, les d'alcools gras issus de l'hydrogénation catalytique des esters méthyliques de coco sont le dodécanol et le décanol.

Dans un mode de réalisation, les alcanes ramifiés de formule I et la comprennent de 30 à 36 atomes de carbones et sont principalement constitués de dimères et de trimères.

Dans un mode de réalisation, les alcanes ramifiés de formule I et Ia sont obtenus par dimérisation successives, par réactions de Guerbet, de dodécanol et décanol, puis déshydratation et hydrogénation.

Dans un mode de réalisation, la dimérisation du décanol donne un octyl-dodécanol, qui peut ensuite être dimérisé avec un dodécanol pour donner un mélange d'isomères en C32, composé majoritairement de trimères. Le milieu peut également comprendre des dimères en C24 issus de la condensation du dodécanol sur lui-même. Le mélange est ensuite soumis à une déshydratation suivie d'une hydrogénation.

Dans un mode de réalisation, les alcanes ramifiés de formule I et la comprennent au moins un alcane trimérique en C30 obtenu par dimérisation de successives de décanol.

Dans un mode de réalisation, les alcanes ramifiés de formule I et la, comprennent au moins un alcane trimérique en C32 obtenu par dimérisations successives de décanol et dodécanol, puis déshydratation et hydrogénation..

Dans un mode de réalisation, les alcanes ramifiés de formule I et Ia comprennent au moins un alcane trimérique en C36 obtenu par dimérisations successives de dodécanol, puis déshydratation et hydrogénation..

Dans un mode de réalisation, n est égal à 1.

Dans un mode de réalisation, n est égal à 0.

Dans un mode de réalisation, les radicaux alkyles sont choisis parmi les radicaux alkyles linéaires en C8 à C12.

Dans un mode de réalisation, l'un au moins des R₁, R'₁, R₂, R'₂ R₃ et R'₃ est un radical alkyle en C8.

Dans un mode de réalisation, l'un au moins des R₁, R'₁, R₂, R'₂ R₃ et R'₃ est un radical alkyle en C9.

Dans un mode de réalisation, l'un au moins des R₁, R'₁, R₂, R'₂ R₃ et R'₃ est un radical alkyle en C10.

Dans un mode de réalisation, l'un au moins des R₁, R'₁, R₂, R'₂ R₃ et R'₃ est un radical alkyle en C11.

Dans un mode de réalisation, l'un au moins des R₁, R'₁, R₂, R'₂ R₃ et R'₃ est un radical alkyle en C12.

Dans un mode de réalisation, l'un au moins des R₁, R'₁, R₂, R'₂ R₃ et R'₃ est un radical alkyle en C13.

Dans un mode de réalisation, l'un au moins des R₁, R'₁, R₂, R'₂ R₃ et R'₃ est un radical alkyle en C14.

Dans un mode de réalisation, l'un au moins des R₁, R'₁, R₂, R_{'2} R₃ et R'₃ R₃ est un radical alkyle en C15.

Dans un mode de réalisation, l'un au moins des R₁, R'₁, R₂, R'₂ R₃ et R'₃ est un radical alkyle en C16.

Dans un mode de réalisation, l'un au moins des R₁, R'₁, R₂, R'₂ R₃ et R'₃ est un radical alkyle en C17.

Dans un mode de réalisation, l'un au moins des R₁, R'₁, R₂, R'₂ R₃ et R'₃ est un radical alkyle en C18.

Dans un mode de réalisation, l'un au moins des R₁, R'₁, R₂, R'₂ R₃ et R'₃ est un radical alkyle en C19.

Dans un mode de réalisation, l'un au moins des R₁, R'₁, R₂, R'₂ R₃ et R'₃ est un radical alkyle en C20.

Dans un mode de réalisation, l'un au moins des R₁, R'₁, R₂, R'₂ R₃ et R'₃ est un radical alkyle en C21.

Dans un mode de réalisation, l'un au moins des R₁, R'₁, R₂, R'₂ R₃ et R'₃ est un radical alkyle en C22.

Dans un mode de réalisation, l'un au moins des R₁, R'₁, R₂, R'₂ R₃ et R'₃ est un radical alkyle en C23.

Dans un mode de réalisation, l'un au moins des R₁, R'₁, R₂, R'₂ R₃ et R'₃ est un radical alkyle en C24.

Dans un mode de réalisation, l'un au moins des R₁, R'₁, R₂, R'₂ R₃ et R'₃ est un radical alkyle en C25.

Dans un mode de réalisation, l'un au moins des R₁, R'₁, R₂, R'₂ R₃ et R'₃ est un radical alkyle en C26.

Dans un mode de réalisation, l'un au moins des R₁, R'₁, R₂, R'₂ R₃ et R'₃ est un radical alkyle en C27.

Dans un mode de réalisation, l'un au moins des R₁, R'₁, R₂, R'₂ R₃ et R'₃ est un radical alkyle en C28.

Dans un mode de réalisation, l'un au moins des R₁, R'₁, R₂, R_{'2} R₃ et R'₃ est un radical alkyle en C29.

Dans un mode de réalisation, l'un au moins des R₁, R'₁, R₂, R_{'2} R₃ et R'₃ est un radical alkyle en C30.
Dans un mode de réalisation, les alcanes trimèriques en C32 sont obtenus par les réactions suivantes :
Etape 1 : dimérisations successives 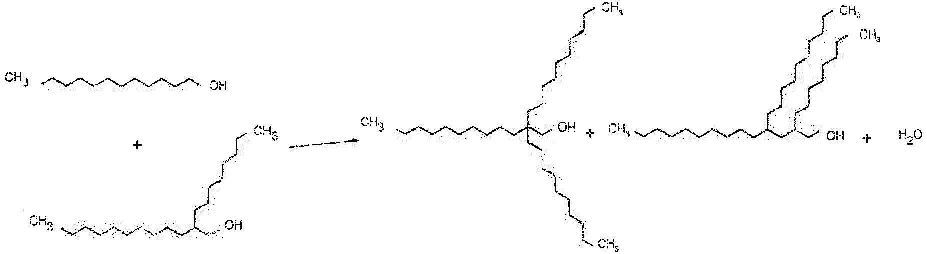
Etape 2 : Déshydratation 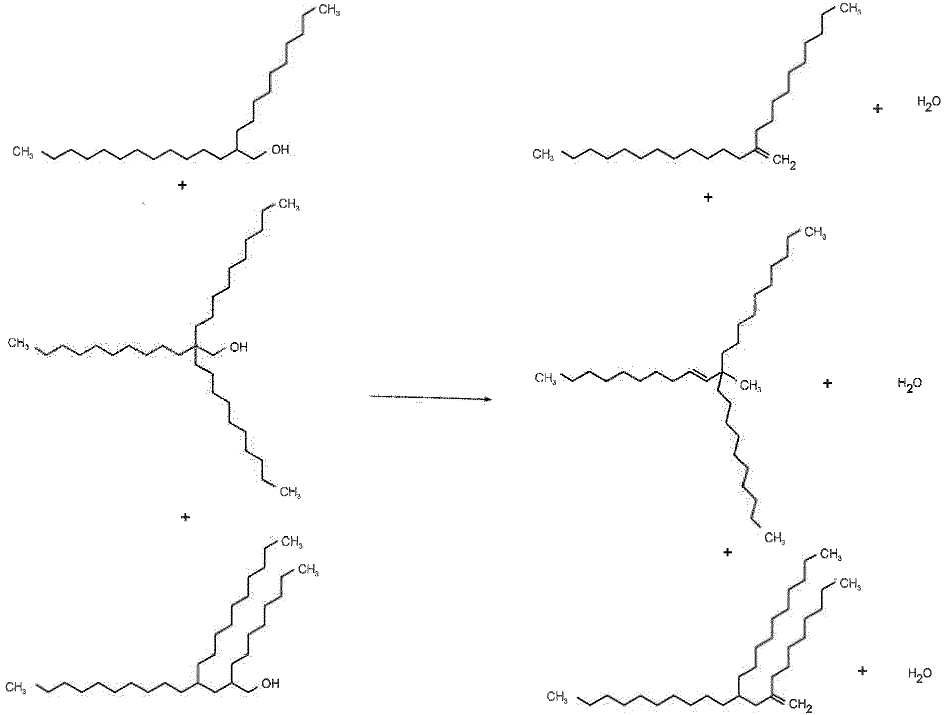
Etape 3 : hydrogénation 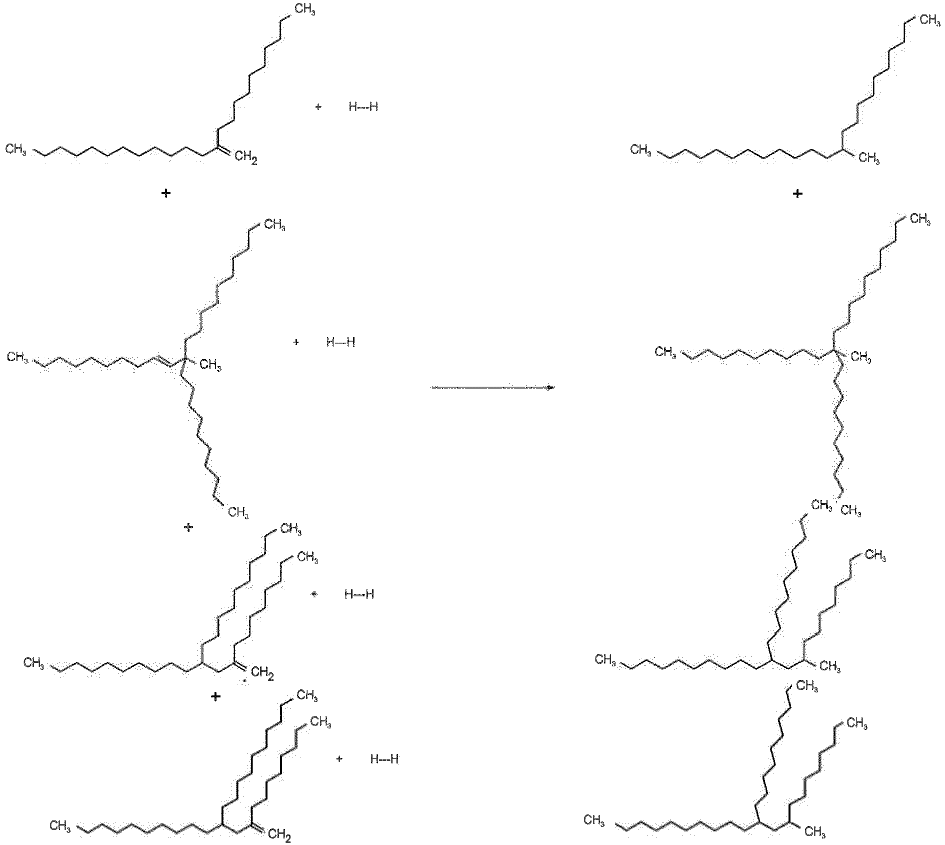
Dans un mode de réalisation, les alcanes trimèriques en C36 sont obtenus par les réactions suivantes :
Etape 1 : dimérisations successives 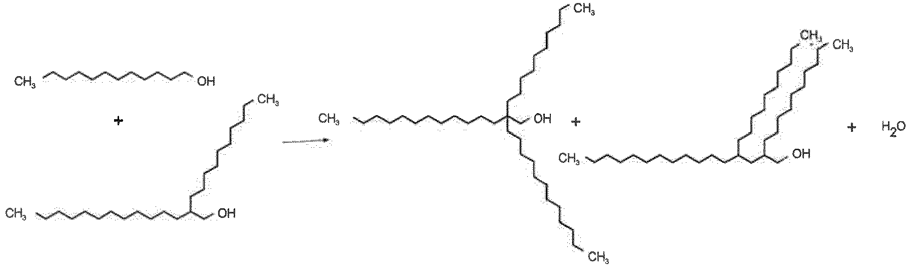
Etape 2 : Déshydratation 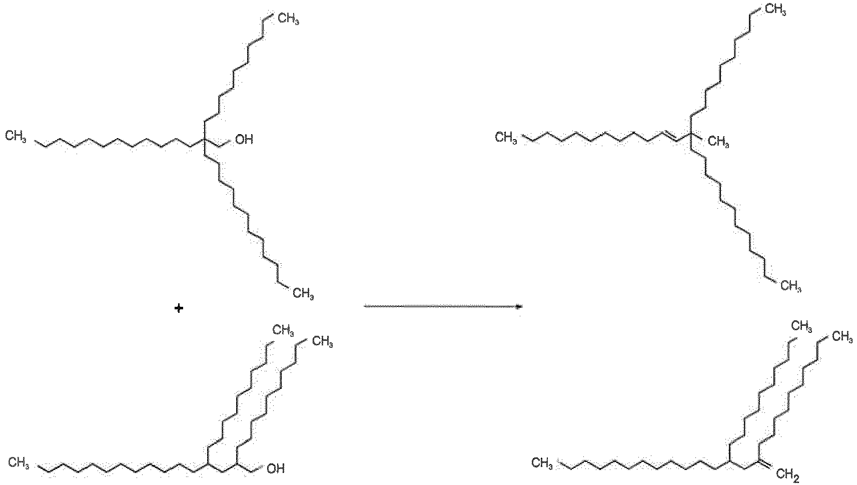
Etape 3 : hydrogénation

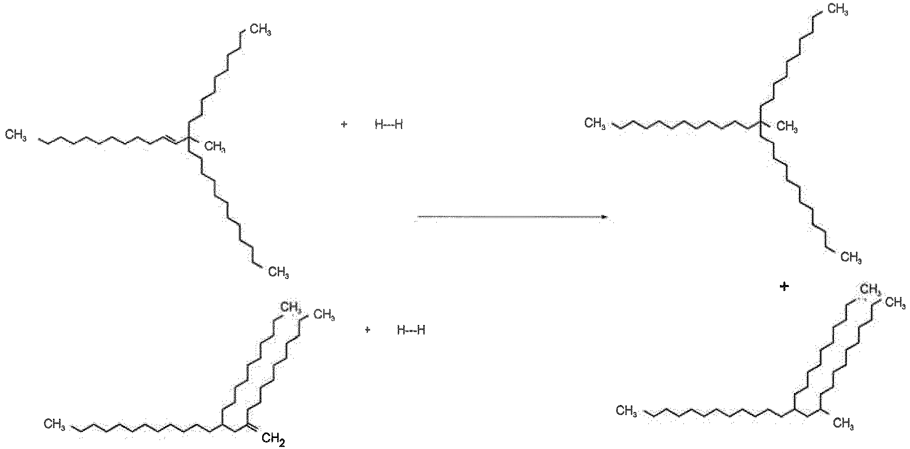

Dans un mode de réalisation, l'ingrédient cosmétique est caractérisé en ce qu'il comprend, en outre, au moins un alcane ramifié en C24 à C48 de formule générale II, 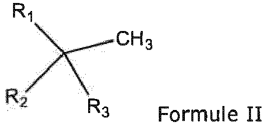 Dans laquelle
- au moins un des R₁, R₂ et R₃ identiques ou différents est choisi parmi les radicaux alkyles, linéaires ou ramifiés en C8 à C30,
et l'un au plus des R₁, R₂ et R₃ est un atome d'hydrogène.
Dans un mode de réalisation, au moins un des R₁, R₂ et R₃ identiques ou différents est choisi parmi les radicaux alkyles linéaires en C8 à C30

Dans un mode de réalisation, l'alcane ramifié comprend de 28 à 36 carbones.

Dans un mode de réalisation, aucun des R₁, R₂ et R₃ est un atome d'hydrogène.

Dans un mode de réalisation, l'un au moins des R₁, R₂ et R₃ est un atome d'hydrogène.

Dans un mode de réalisation, l'un au moins des R₁, R₂ et R₃ est un radical alkyle en C8.

Dans un mode de réalisation, l'un au moins des R₁, R₂ et R₃ est un radical alkyle en C9.

Dans un mode de réalisation, l'un au moins des R₁, R₂ et R₃ est un radical alkyle en C10.

Dans un mode de réalisation, l'un au moins des R₁, R₂ et R₃ est un radical alkyle en C11.

Dans un mode de réalisation, l'un au moins des R₁, R₂ et R₃ est un radical alkyle en C12.

Dans un mode de réalisation, l'un au moins des R₁, R₂ et R₃ est un radical alkyle en C13.

Dans un mode de réalisation, l'un au moins des R₁, R₂ et R₃ est un radical alkyle en C14.

Dans un mode de réalisation, l'un au moins des R₁, R₂ et R₃ est un radical alkyle en C15.

Dans un mode de réalisation, l'un au moins des R₁, R₂ et R₃ est un radical alkyle en C16.

Dans un mode de réalisation, l'un au moins des R₁, R₂ et R₃ est un radical alkyle en C17.

Dans un mode de réalisation, l'un au moins des R₁, R₂ et R₃ est un radical alkyle en C18.

Dans un mode de réalisation, l'un au moins des R₁, R₂ et R₃ est un radical alkyle en C19.

Dans un mode de réalisation, l'un au moins des R₁, R₂ et R₃ est un radical alkyle en C20.

Dans un mode de réalisation, l'un au moins des R₁, R₂ et R₃ est un radical alkyle en C21.

Dans un mode de réalisation, l'un au moins des R₁, R₂ et R₃ est un radical alkyle en C22.

Dans un mode de réalisation, l'un au moins des R₁, R₂ et R₃ est un radical alkyle en C23.

Dans un mode de réalisation, l'un au moins des R₁, R₂ et R₃ est un radical alkyle en C24.

Dans un mode de réalisation, l'un au moins des R₁, R₂ et R₃ est un radical alkyle en C25.

Dans un mode de réalisation, l'un au moins des R₁, R₂ et R₃ est un radical alkyle en C26.

Dans un mode de réalisation, l'un au moins des R₁, R₂ et R₃ est un radical alkyle en C27.

Dans un mode de réalisation, l'un au moins des R₁, R₂ et R₃ est un radical alkyle en C28.

Dans un mode de réalisation, l'un au moins des R₁, R₂ et R₃ est un radical alkyle en C29.

Dans un mode de réalisation, l'un au moins des R₁, R₂ et R₃ est un radical alkyle en C30.

Dans un mode de réalisation, le au moins alcane ramifié de formule II est obtenu par déshydratation d'un alcool de Guerbet en C24 à C48 de formule générale III, 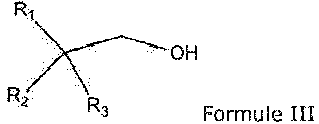 dans laquelle R₁, R₂ et R₃ ont les valeurs définies ci-dessus, suivie d'une hydrogénation.

Dans un mode de réalisation, les au moins alcanes ramifiés en C36 sont obtenus par Déshydratation de l'Isofol 36 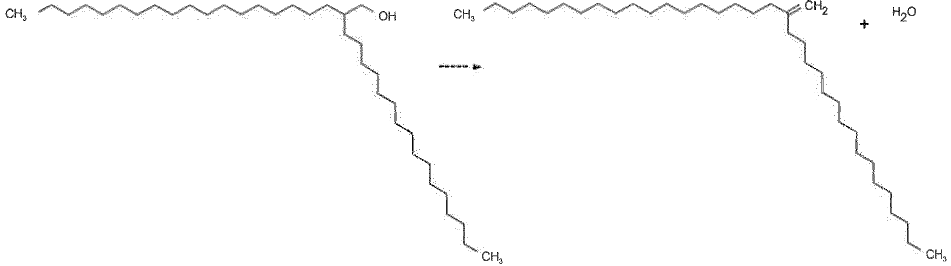 suivie d'une hydrogénation, pour obtenir le 17-methyl pentatriacontane. Dans un mode de réalisation, l'ingrédient cosmétique peut en outre comprendre des alcanes en C18 à C28 de formule générale I, obtenus par déshydrogénation d'alcools issus de sources végétales.

Les dimérisations sont effectuées par réaction de Guerbet, à parti d'alcools linéaires ; on obtient des alcools ramifiés en position 2. Les réactions de Guerbet sont mises en œuvre en présence d'hydroxides ou d'alkoxides métalliques et de catalyseurs du type nickel de Raney à des températures supérieures à 220°C et sous pression. Des conditions de réactions telles que celles décrites dans le brevet US 2003/0181770 au nom de COGNIS peuvent également être mises en œuvre.

Les alcools de Guerbet ou au moins dimèriques mis en œuvre peuvent également être achetés, par exemple à la société SASOL.

Les réactions de déshydratation sont mises en œuvre selon des méthodes classiques à haute température en présence d'alumine ou selon des méthodes décrites par exemple dans la demande de brevet WO2010121591 au nom de SASOL. Dans une autre variante de l'invention, les alcanes peuvent être obtenus à partir des alcools gras par hydrogénolyse de la fonction hydroxyle, en présence d'hydrogène et d'un catalyseur métallique.

Les réactions d'hydrogénation sont mises effectuées par mise en œuvre de catalyseurs classiques comme des catalyseurs au nickel commercialisés par la société Johnson Matthey ou par des catalyseurs de nouvelle génération au Palladium comme ceux décrits dans la publication de R. Ciriminna et al., dans Org. Process Res. Dev.,2014,18(9), pp1110-1115.

L'invention concerne également l'utilisation de l'ingrédient cosmétique selon l'invention, en association avec un squalane végétal pour donner une composition dont les propriétés organoleptiques et sensorielles sont conservées tout en utilisant moins de squalane végétal.

Elle concerne l'utilisation de l'ingrédient cosmétique selon l'invention, en association avec un squalane végétal pour préparer une composition dont les propriétés organoleptiques et sensorielles sont conservées tout en substituant au moins 50 % de squalane végétal dans la composition.

L'invention concerne également une composition contenant au plus 50 % de squalane végétale en association avec l'ingrédient cosmétique selon l'invention.

L'invention concerne également une composition comprenant au plus 50 % de squalane végétal en association avec l'ingrédient cosmétique selon l'invention.

L'invention concerne également une composition comprenant de 30 à 50 % de squalane végétal en association avec 50 à 70 % de l'ingrédient cosmétique selon l'invention.

### Exemples

### I - Conditions générales :

### Réaction de dimérisation :

La réaction de Guerbet entre deux alcools identiques ou différents donne un mélange composé de différents isomères. Cette synthèse est réalisée en présence d'un mélange de deux alcools, de préférence à ratio équimolaire et en présence d'une base alcaline et d'un catalyseur à base d'oxyde de cuivre.

### Procédé de déshydratation :

Le réacteur utilisé est un réacteur tubulaire continu qui présente les caractéristiques suivantes :
▪ Diamètre intérieur : 12,5 mm
▪ Hauteur totale : 370 mm
▪ Hauteur du lit de catalyseur : 100 mm

Le lit de catalyseur est placé à la mi-hauteur du réacteur. Un thermocouple est placé au cœur de ce lit. Ce dernier est maintenu par une grille sur laquelle est placée de la laine de silice. Au-dessus du lit, de la laine de silice est ajoutée afin de bloquer le lit de catalyseur.

Pour cette étape de déshydratation, le catalyseur utilisé est une alumine industrielle (Al₂O₃ 99 %).

Une masse de 6,436 g a été chargée dans le réacteur, soit un volume de 11,9 mL.

### Réactions d'hydrogénation

Les réactions d'hydrogénation se déroulent dans un réacteur batch d'un litre de capacité. Les conditions opératoires sont détaillées ci-dessous.

L'alcène et le catalyseur (nickel de Raney) sont introduits dans le réacteur à température ambiante. Le réacteur est inerté par 3X5 bars d'azote et la température est élevée à la température de travail de 180°C. 5 bars de dihydrogène sont ensuite introduits. Au bout de deux heures la température est élevée à 200°C. la réaction est ensuite maintenue 3h supplémentaires.

### II Obtention des alcanes sous forme au moins dimérique

### II-1 Obtention des alcanes sous forme au moins dimérique en C30

Réaction de Guerbet entre le décanol et l'octyldodécanol (obtenu par dimérisation du décanol). Le décanol est un alcool gras issu de l'hydrogénation catalytique des esters méthyliques de coco.

La réaction recherchée entre le décanol et l'octyldodécanol donne un mélange d'isomères en C30 composé majoritairement de trimères. Voir schémas ci-dessus. Elle peut également donner lieu à la formation de dimères en C20 issus de la réaction du décanol sur lui-même.

Le mélange d'alcools est ensuite déshydraté selon les conditions générales décrites ci-dessus.

Le mélange d'alcènes obtenus est ensuite hydrogéné, pour obtenir des alcanes trimèriques en C30 en moyenne, selon l'invention.

| Consommation de dihydrogène pour 1 Tonne d'alcène, kg H₂ | Formule alcane moyenne) | Indice d'iode alcène g I2/100g | Viscosité alcane 40°C, mm²/s | Point d'écoulement alcane, °C |
|---|---|---|---|---|
| 4,8 | C30H62 | < 1 | 14 | - 54 |

### II-2 Obtention des alcanes sous forme au moins dimérique en C32

Réaction de Guerbet entre le dodécanol et l'octyldodécanol (obtenu par dimérisation du décanol). Le dodécanol et le décanol sont des alcools gras issus de l'hydrogénation catalytique des esters méthyliques de coco.

La réaction recherchée entre le dodécanol et l'octyldodécanol donne un mélange d'isomères en C32 composé majoritairement de trimères. Elle peut également donner lieu à la formation de dimères en C24 issu de la réaction du dodécanol sur lui-même.

Le mélange d'alcools est ensuite déshydraté selon les conditions générales décrites ci-dessus.

Le mélange d'alcènes obtenus est ensuite hydrogéné, pour obtenir des alcanes trimèriques en C32 en moyenne, selon l'invention.

| Consommation de dihydrogène pour 1 Tonne d'alcène, kg H₂ | Formule alcane) (moyenne) | Indice d'iode alcène g I2/100g | Viscosité alcane 40°C, mm²/s | Point d'écoulement alcane, °C |
|---|---|---|---|---|
| 4,5 | C32H66 | < 1 | 16 | - 30 |

### II-3 Obtention des alcanes sous forme au moins dimérique en C36

Réaction de Guerbet entre le dodécanol et le décatétradécanol (obtenu par dimérisation du dodécanol). Le dodécanol est un alcool gras issu de l'hydrogénation catalytique des esters méthyliques de coco.

La réaction recherchée entre le dodécanol et le décatétradécanol donne un mélange d'isomères en C36 composé majoritairement de trimères. Elle peut également donner lieu à la formation de dimères en C24 issu de la réaction du dodécanol sur lui-même.

Le mélange d'alcools est ensuite déshydraté selon les conditions générales décrites ci-dessus.

Le mélange d'alcènes obtenus est ensuite hydrogéné, pour obtenir des alcanes trimèriques en C36 en moyenne, selon l'invention.

| Consommation de dihydrogène pour 1 Tonne d'alcène, kg H₂ | Formule alcane (moyenne) | Indice d'iode alcène g I2/100g | Viscosité alcane 40°C, mm²/s | Point d'écoulement alcane , °C |
|---|---|---|---|---|
| 4,0 | C36H74 | < 1 | 20 | - 24 |

III Exemples de synthèse des alcanes ramifiés de formules générales II :

### III-1 Déshydratation

Les matières premières utilisées sont les suivantes :

| Matières première | Référence | Fournisseur |
|---|---|---|
| 2-decyltetradécanol | Isofol 24 | SASOL |
| 2-tetradecyloctadeacanol | Isolfol 32 | SASOL |
| 2-hexadécyleicosanol | Isofol 36 | SASOL |
| Alumine | | INDUSTRIELLE 99% |
| Laine de silice | | VWR |
| Azote | Industriel | AIR LIQUIDE |

Le LHSV (Liquid Hourly Space Velocity) correspond au débit d'alcool passé au travers du réacteur exprimé en mL/min/mL de catalyseur.

**Résultats deshydratation**

| Alcool | T, °C | LHSV | d (mL/min) | Conversion | Quantité d'eau produite pour 1 tonne d'alcool, kg | Quantité d'alcene produite pour 1 tonne d'alcool, kg |
|---|---|---|---|---|---|---|
| Isofol 24 | 330 | 2,5 | 0,5 | >99% | 50,8 | 949,2 |
| Isofol 32 | 330 | 2,5 | 0,5 | >99% | 38,6 | 961,4 |
| Isofol 36 | 330 | 2,5 | 0,5 | >99% | 34,5 | 965,5 |

**Caractérisation alcènes**

| Alcène | Formule Alcène | Indice d'iode alcène g I₂/100g |
|---|---|---|
| Alcène iC24 | C24H48 | 75,5 |
| Alcène iC32 | C32H64 | 56,7 |
| Alcène iC36 | C36H72 | 50,4 |
| Squalène végétal | C30H50 | 371,4 |
| Squalène marin | C30H50 | 371,4 |

### III-2 Hydrogénation

Selon le mode opératoire décrit ci-dessus partie I-3 on obtient les alcanes dont les caractéristiques sont données dans la tableau ci-dessous.

**Caractérisation des alcanes**

| Alcane | Consommation d'hydrogène pour 1 Tonne d'alcène, kg H2 | Formule alcane | Viscosité 40°C, mm²/s | Aspect à température ambiante |
|---|---|---|---|---|
| Alcane i C24 | 6,0 | C24H50 | 8,0 | Liquide |
| Alcane i C32 | 4,5 | C32H66 | Nd | Solide |
| Alcane i C36 | 4,0 | C36H74 | nd | Solide |
| Squalane végétal | 29,3 | C30H62 | 21,7 | Liquide |
| Squalane marin | 29,3 | C30H62 | 16,6 | Liquide |

### IV- Exemples de compositions selon l'invention

### Composition R

Une composition selon l'invention est préparée en mélangeant :
- 50 % de squalane d'olive,
- 35 % d'alcane sous forme au moins dimérique en C36,
- 10 % d'alcane sous forme au moins dimérique en C30
- 5 % d'alcane ramifié en C32.

On obtient une composition qui est non grasse, fine et filmogène.

La stabilité est équivalente à celle du squalane de requin.

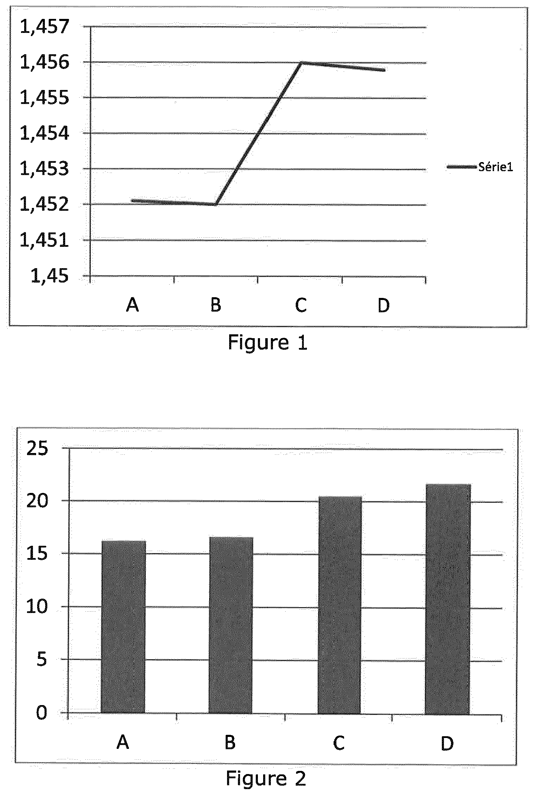

Les viscosités à 40°C en mm2/s et les indices de réfractions sont comparés dans les tableaux ci-après et les figures 1 et 2, à ceux du squalane d'olive, du squalane de canne à sucre, du squalane de requin.
Indices de réfraction (voir Figure 1)

| Nomenclature produit | Produit testé | Indice de réfraction |
|---|---|---|
| A | Squalane de canne à sucre | 1,4521 |
| B | Squalane de requin | 1,4520 |
| C | Squalane d'olive | 1,4560 |
| D | Composition selon l'invention | 1,4558 |

Viscosités à 40°C en mm²/s (voir Figure 2)

| Nomenclature produit | Produit testé | Viscosité à 40°C en mm²/s |
|---|---|---|
| A | Squalane de canne à sucre | 16,2 |
| B | Squalane de requin | 16,6 |
| C | Squalane d'olive | 20,5 |
| D | Composition selon l'invention | 21,7 |

En conclusion, les viscosités et les indices de réfraction de la composition selon l'invention sont équivalents à ceux du squalane d'olive.

### Composition S

Une composition selon l'invention est préparée en mélangeant :
- 30 % de squalane d'olive,
- 17,5 % d'alcane sous forme au moins dimérique en C24,
- 52,5 % d'alcane sous forme au moins dimérique en C36,

### Composition T

Une composition selon l'invention est préparée en mélangeant :
- 50 % de squalane d'olive,
- 10 % d'alcane sous forme au moins dimérique en C20,
- 40 % d'alcane sous forme au moins dimérique en C36,

### V- Caractérisation par texturométrie des compositions selon l'invention

Un test reproduisant les caractéristiques sensorielles a été effectué et la consistance, la cohésion, la fermeté et le collant ont été caractérisés pour des compositions selon l'invention et des squalanes.

Les protocoles utilisés sont décrits dans la thèse de Laura Gilbert. Caractérisation physico-chimique et sensorielle d'ingrédients cosmétiques : une approche méthodologique. Polymers. Université du Havre, 2012, notamment page 126 et 127 et dans la publication de Laura Gilbert dans 2 Colloids and surfaces A : Physicochem. Eng. Aspects 421(2013) 150-163 page 152 paragraphe 2.2.3.

L'appareil utilisé est un texturomètre TA.XT Plus.

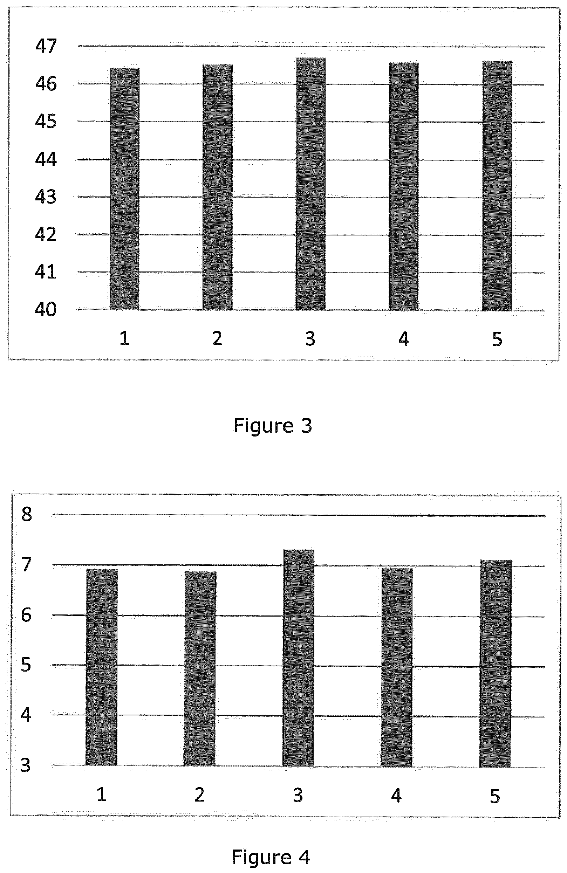

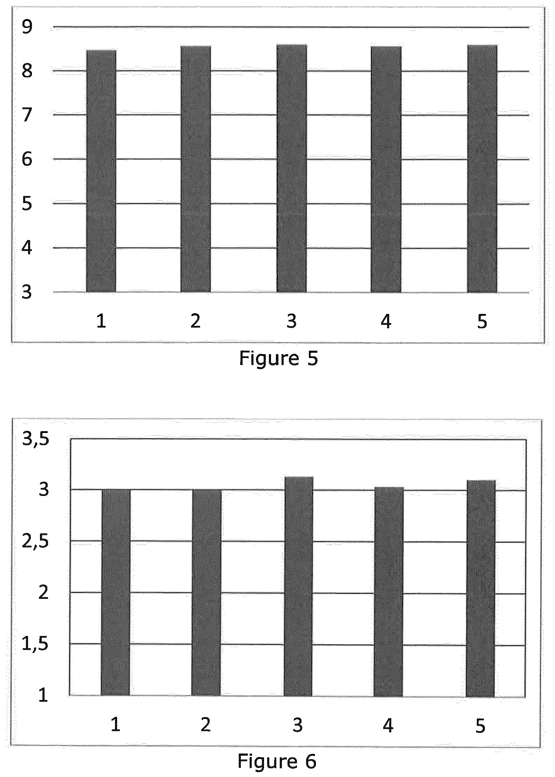

Dans les Figures 3, 4, 5 et 6 les résultats relatifs aux compositions ou ingrédients suivants ont été rassemblés :
- Produit 1 : Sugarcane squalane Neossance
- Produit 2 :Plantasens OLIVE Squalane de Clariant
- Produit 3 : Olive squalane (Squalive) Biosynthis
- Produit 4 : 50% mélange d'alcanes en C20 à C36 + 50% Olive squalane (Squalive) Biosynthis (composition S)
- Produit 5 : 70% mélange d'alcanes en C24 à C36 + 30% Olive squalane (Squalive) Biosynthis (composition r)

La figure 3 représente les résultats obtenus en consistance en g.s. Les résultats sont soumis à une analyse de variance (ANOVA) et aucune différence significative entre les différents produits testés n'est observée.

La figure 4 représente les résultats obtenus en cohésion en g.s. Les résultats sont soumis à une analyse de variance (ANOVA) et aucune différence significative entre les différents produits testés n'est observée.

La figure 5 représente les résultats obtenus en fermeté en g. Les résultats sont soumis à une analyse de variance (ANOVA) et aucune différence significative entre les différents produits testés n'est observée.

La figure 6 représente les résultats obtenus en collant en g. Les résultats sont soumis à une analyse de variance (ANOVA) et aucune différence significative entre les différents produits testés n'est observée.

### VI- Caractérisation par goniométrie des compositions selon l'invention

Des compostions selon l'invention et des squalanes ont été caractérisés par goniométrie selon le protocole décrit dans la publication de G. Savary dans Colloids and surfaces Biointerfaces 102 (2013) 371-378 à la page372 paragraphe 2.2.1.

L'appareil utilisé est un Digidrop GBX.

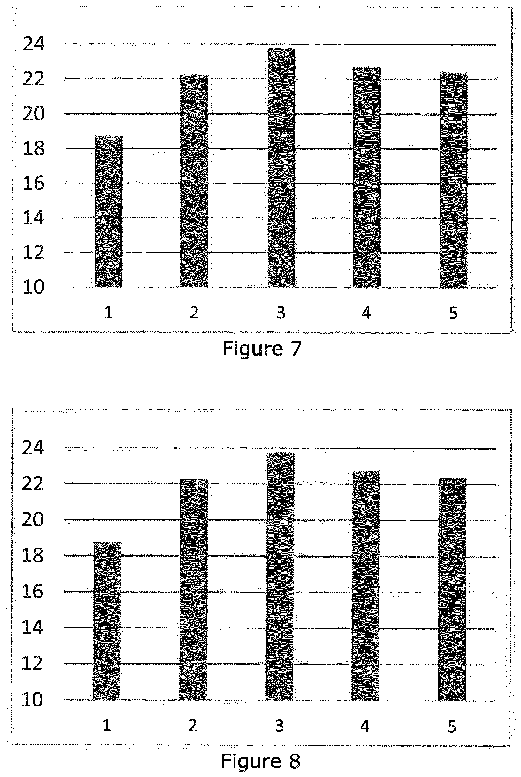

Dans les Figures 7 et 8 les résultats relatifs aux compositions ou ingrédients suivants ont été rassemblés :
- Produit 1 : Sugarcane squalane Neossance
- Produit 2 :Plantasens OLIVE Squalane de Clariant
- Produit 3 : Coco squalane (Squalive) Biosynthis
- Produit 4 : 50% mélange d'alcanes en C24 à C48 + 50% Coco squalane (Squalive) Biosynthis
- Produit 5 : 70% mélange d'alcanes en C24 à C48 + 30% Coco squalane (Squalive) Biosynthis

Les résultats sont exprimés en degré ° et sont soumis à une analyse de variance (ANOVA)

Les résultats présentés à la Figure 7 sont des mesures d'angle de contact après 1 s.

Les résultats présentés à la Figure 8 sont des mesures d'angle de contact après 4 s.

### V- Formulations cosmétiques comprenant des compositions selon l'invention

### Composition de fond de teint :

**Phase A :**

| INCI | Ingrédients | % en masse/masse totale |
|---|---|---|
| | Cétyl PEG/PPG-10/1 diméthicone | 2,80 |
| Abil EM 90 | Phényltrimethicone | 1 |
| | Composition selon l'invention S | 3 |
| | Cétéaryl ethylhexanoate | 2 |
| Vegelight 1214 | Hydrocarbures végétaux | 1,8 |

**Phase B :**

| Ingrédients | % en masse/masse totale |
|---|---|
| Mica | 0,5 |
| Red oxide et diméthicone | 0,22 |
| Yellow oxide et diméthicone | 0,75 |
| Black iron oxide et diméthicone | 0,12 |
| Titanium oxide et diméthicone | 8,5 |

**Phase C**

| Ingrédients | % en masse/masse totale |
|---|---|
| Sodium chloride | 1,25 |
| Phenoxyéthanol | 0,50 |
| Butylène glycol | 5,00 |
| Eau | Qsp. 100 |
| Parfum | q.s. |

### Composition d'huile sèche

**Phase A :**

| Ingrédients | INCI | % en masse/masse totale |
|---|---|---|
| Caprylic/cpric triglycéride | Neoderm MCT | 10,00 |
| *Simmondsia Chinensis* | Organic Jojoba oil | 5,00 |
| Composition selon l'invention S | | 59,9 |

**Phase B :**

| Ingrédients | INCI | % en masse/masse totale |
|---|---|---|
| Hydrocarbures végétaux | VEGELIGHT 1214 | 20,00 |
| Parfum | | q.s. |

## Revendications

1. Ingrédient cosmétique comprenant au moins un squalane végétal et au moins un mélange d'alcanes en C24 à C48, sous forme au moins dimérique, choisis parmi les alcanes de formule I, 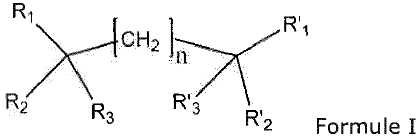 dans laquelle
- n est 1 ou 0,
- R₁, R'₁, R₂, R'₂, R₃ et R'₃ identiques ou différents sont choisis dans le groupe constitué par les atomes d'hydrogène -H, les radicaux méthyles et les radicaux alkyles linéaires en C8 à C30,
- au moins un des R₁, R'₁, R₂, R'₂, R₃ et R'₃ identiques ou différents est choisi parmi les radicaux alkyles linéaires en C8 à C30,
et l'un au moins et l'un au plus des R₁, R'₁, R₂, R_{'2} R₃ et R'₃ est un radical méthyle, ledit mélange d'alcanes en C24 à C48 ayant une viscosité comprise entre 12 et 25 mm²/s à 40°C.

2. Ingrédient cosmétique selon la revendication 1, **caractérisé en ce que** les alcanes sont choisis parmi les alcanes de formule I, dans laquelle R'₁, R₁ et R₂ sont des atomes d'hydrogène, R'₂ est un radical méthyle et n est égal à 0, de formule la, dans laquelle, R3 et R'3 sont choisis parmi les radicaux alkyles linéaires en C8 à C30

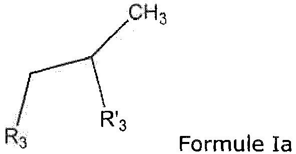

3. Ingrédient selon l'une quelconque des revendications précédentes **caractérisé en ce que** les alcanes ramifiés de formule I ou Ia comprennent de 30 à 36 atomes de carbones et sont principalement constitués de dimères et de trimères.

4. Ingrédient selon la revendication 1, **caractérisé en ce que** n est égal à 1.

5. Ingrédient selon la revendication 1, **caractérisé en ce que** n est égal à 0

6. Ingrédient selon l'une quelconque des revendications 1, 2, 3, 4 ou 5, **caractérisé en ce que** les radicaux alkyles linéaires sont identiques et choisis parmi les radicaux alkyles linéaires en C8 à C12

7. Ingrédient selon l'une quelconque des revendications précédentes **caractérisé en ce qu'**il comprend, en outre, au moins un alcane ramifié en C24 à C48 de formule générale II, 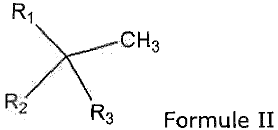 dans laquelle
- au moins un des R₁, R₂ et R₃ identiques ou différents est choisi parmi les radicaux alkyles, linéaires en C8 à C30, et l'un au plus des R₁, R₂ et R₃ est un atome d'hydrogène.

8. Ingrédient selon la revendication 7, **caractérisé en ce que** le au moins alcane ramifié de formule II comprend de 28 à 36 carbones.

9. Utilisation de l'ingrédient cosmétique selon l'une quelconque des revendications précédentes, pour préparer une composition dont les propriétés organoleptiques et sensorielles sont conservées tout en substituant au moins 50 % de squalane végetal dans la composition.

## Patentansprüche

1. Kosmetischer Bestandteil umfassend mindestens ein Pflanzensqualan und wenigstens eine Mischung aus Alkanen mit C24- bis C48, mindestens in dimerer Form, ausgewählt aus den Alkanen gemäß Formel I, 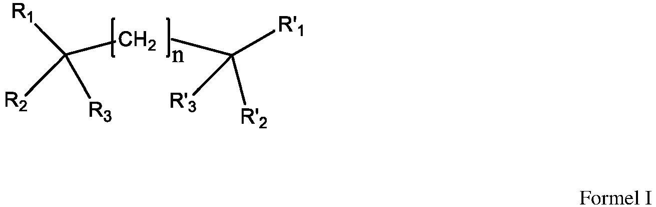 wobei
- n 1 oder 0 ist,
- R₁, R'₁, R₂, R'₂, R₃ und R'₃, die identisch oder verschieden sind, ausgewählt sind aus der Gruppe bestehend aus Wasserstoffatomen -H und linearen Methylradikalen mit C8 bis C30,
- wenigstens einer von R₁, R'₁, R₂, R'₂, R₃ und R'₃, die identisch oder verschieden sind, ausgewählt ist aus den linearen Alkylradikalen mit C8 bis C30,
- und wenigstens einer und höchstens einer von R₁, R'₁, R₂, R'₂, R₃ und R'₃ ein Methylradikal ist, wobei die Mischung aus Alkanen mit C24 bis C48 eine Viskosität von 12 bis 25 mm²/s bei 40°C aufweist.

2. Kosmetischer Bestandteil nach Anspruch 1, **dadurch gekennzeichnet, dass** die Alkane ausgewählt sind aus den Alkanen gemäß Formel I, wobei R'₁, R₁ und R₂ Wasserstoffatome sind, R'₂ ein Methylradikal und n gleich 0 ist, gemäß Formel Ia, wobei R₃ und R'₃ ausgewählt sind aus den linearen Alkylradikalen mit C8 bis C30

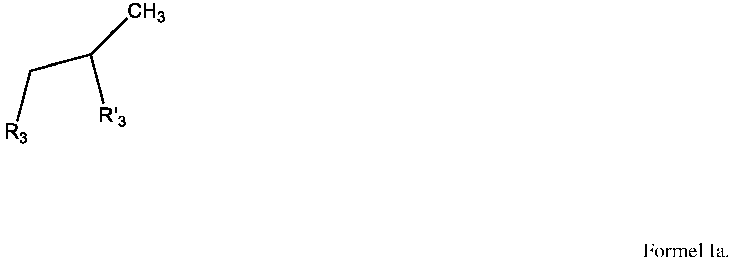

3. Bestandteil nach einem der vorangehenden Ansprüche, **dadurch gekennzeichnet, dass** die verzweigten Alkane gemäß Formel I oder Ia 30 bis 36 Kohlenstoffatome umfassen und vorwiegend aus Dimeren und Trimeren bestehen.

4. Bestandteil nach Anspruch 1, **dadurch gekennzeichnet, dass** n gleich 1 ist.

5. Bestandteil nach Anspruch 1, **dadurch gekennzeichnet, dass** n gleich 0 ist.

6. Bestandteil nach einem der Ansprüche 1, 2, 3, 4 oder 5, **dadurch gekennzeichnet, dass** die linearen Alkylradikale identisch sind und ausgewählt sind aus den linearen Alkylradikalen mit C8 bis C12.

7. Bestandteil nach einem der vorangehenden Ansprüche, **dadurch gekennzeichnet, dass** er, außerdem, wenigstens ein verzweigtes Alkan mit C24 bis C48 gemäß der allgemenen Formel II umfasst 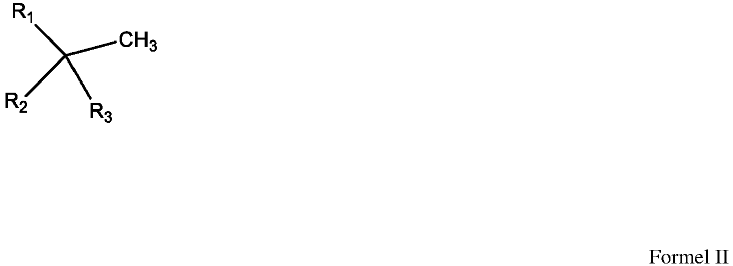 wobei wenigstens einer von R₁, R₂ und R₃, die identisch oder verschieden sind, ausgewählt ist aus den linearen Alkylradikalen mit C8 bis C30, und höchstens einer von R₁, R₂ und R₃ ein Wasserstoffatom ist.

8. Bestandteil nach Anspruch 7, **dadurch gekennzeichnet, dass** das wenigstens eine verzweigte Alkan gemäß Formel II 28 bis 36 Kohlenstoffatome umfasst.

9. Verwendung eines kosmetischen Bestandteils nach einem der vorangehenden Ansprüche, zum Herstellen einer Zusammensetzung, deren organoleptischen und sensorischen Eigenschaften beibehalten bleiben, während wenigstens 50% Pflanzensqualan in der Zusammensetzung ersetzt sind.

## Claims

1. A cosmetic ingredient comprising at least one plant squalane and at least one mixture of C24 to C48 alkanes, in at least dimeric form, selected from the alkanes of formula I. 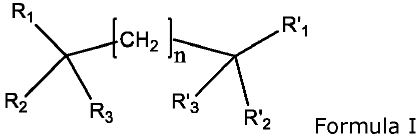 wherein
- n is 1 or 0,
- R₁, R'₁, R₂, R'₂, R₃ and R'₃ are the same or different and selected from the group consisting of hydrogen atoms -H, methyl radicals and linear C8 to C30 alkyl radicals,
- at least one of R₁, R'₁, R₂, R'₂, R₃ and R'₃ identical or different, is selected from the linear C8 to C30 alkyl radicals,
and at least one and at most one of R₁, R'₁, R₂, R'₂ R₃ et R'₃ is a methyl radical, said mixture of C24 to C48 alkanes having a viscosity of between 12 and 25 mm²/s at 40°C.

2. A cosmetic ingredient according to claim 1, **characterized in that** the alkanes are chosen from alkanes of formula I, in which R'₁, R₁ et R₂ are hydrogen atoms, R'₂ is a methyl radical and n is equal to 0, of formula Ia, in which, R₃ et R'₃ are chosen from linear C8 to C30 alkyl radicals.

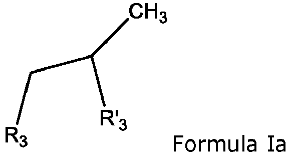

3. Ingredient according to any of the preceding claims **characterized in that** the branched alkanes of formula I or Ia comprise 30 to 36 carbon atoms and are mainly composed of dimers or trimers.

4. Ingredient according to claim 1, **characterized in that** n is equal to 1.

5. Ingredient according to claim 1, **characterized in that** n is equal to 0.

6. Ingredient according to any one of claims 1, 2, 3, 4 or 5, **characterized in that** the linear alkyl radicals are identical and are selected from linear C8 to C12 alkyl radicals.

7. Ingredient according to any of preceding claims, **characterized in that** it further comprises at least one branched C24 to C48 alkane of general formula II, 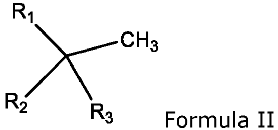 wherein
- at least one of R₁, R₂ and R₃ identical or different, is selected from the linear C8 to C30 alkyl radicals,
and at most one of R₁, R₂ and R₃ is an hydrogen atom.

8. Ingredient according to claim 7, **characterized in that** the at least one branched alkane of formula II comprises 28 to 36 carbons.

9. Use of the cosmetic ingredient according to any one of the preceding claims for preparing a composition whose organoleptic and sensory properties are preserved while substituting at least 50% of plant squalane in the composition.
